# FASCICULE DE BREVET EUROPEEN

(11) **EP 2 699 890 B1**
(45) Date de publication et mention de la délivrance du brevet: **25.11.2015**
(21) Numéro de dépôt: 12722535.7
(22) Date de dépôt: 18.04.2012
(51) Int. Cl.: G01N 21/35, G02B 6/122, G02B 6/10, G02B 6/12

(54) **DISPOSITIF D'EMISSION ET DE GUIDAGE D'UN RAYONNEMENT INFRAROUGE**
VORRICHTUNG ZUR EMISSION UND LEITEN VON INFRAROTSTRAHLUNG
DEVICE FOR EMITTING AND GUIDING AN INFRARED RADIATION

(30) Priorité: 19.04.2011 FR 1153380
(43) Date de publication de la demande: 26.02.2014
(73) Titulaire: Commissariat à l'Énergie Atomique et aux Énergies Alternatives, 75015 Paris (FR)
(72) Inventeur: GIDON, Serge, F-38140 La Murette (FR)
(74) Mandataire: Corret, Hélène
(86) Numéro de dépôt international: PCT/IB2012/051947
(87) Numéro de publication internationale: WO 2012/143869

(56) Documents cités:
- WO-A1-00/04616
- US-A- 5 834 777
- US-A1- 2007 120 057
- US-A1- 2011 073 875
- US-B1- 7 418 166

## Description

La présente invention concerne un dispositif d'émission et de guidage d'un rayonnement infrarouge.

Des dispositifs de ce type ont déjà été proposés. Ils comportent, de manière générale, un guide d'onde et des moyens d'émission d'un rayonnement infrarouge.

La difficulté principale de ces dispositifs est d'assurer un couplage efficace entre le guide d'onde et les moyens d'émission.

On peut ainsi citer l'article de J. Kasberger et B. Jakoby, « Grating-coupling of thermal radiation as an essential element of a fully integrated IR-absorption sensor system » paru dans OPTO Conférence 2008 Proceedings, pages 161 et suivantes.

Cet article décrit un capteur dont le fonctionnement est basé sur l'absorption infrarouge dans la région de champ évanescent d'un guide d'onde utilisant une radiation infrarouge générée thermiquement. Le couplage entre le radiateur thermique émettant la radiation infrarouge et le guide d'onde est réalisé par l'intermédiaire d'un coupleur à réseau.

Cependant, l'efficacité du couplage obtenu reste modeste. Le document US 5 834 777 décrit un capteur avec une source thermique couplée à un guide d'onde par un prisme.

Il convient par ailleurs de noter que certaines solutions, connues dans le domaine de l'optique macroscopique, ne peuvent pas être transposées à des guides d'onde.

Ainsi, en photoacoustique notamment, on peut utiliser des sources thermiques réalisées sous forme de filaments. Cependant, elles ne peuvent pas être utilisées avec un guide d'onde, du fait de leur grande divergence.

C'est pourquoi la solution qui est couramment retenue consiste à coupler le guide d'onde à une source laser. Ainsi, des sources laser ont déjà été réalisées sur la base de structures semi-conductrices complexes.

Elles restent cependant difficiles à maîtriser et, à ce jour, leur rendement énergétique est assez faible, puisqu'il est de l'ordre de 1/1 000.

L'invention a pour objet de pallier ces inconvénients en proposant un dispositif d'émission et de guidage d'un rayonnement infrarouge assurant un couplage efficace entre le guide d'onde et les moyens d'émission d'un rayonnement infrarouge. L'invention est définie dans la revendication 1.

Selon l'invention, ce dispositif est caractérisé en ce que lesdits moyens d'émission d'un rayonnement infrarouge peuvent être formés en surface ou à l'intérieur du guide d'onde.

La présence des moyens d'émission en surface ou à l'intérieur du guide d'onde permet une transmission efficace d'un rayonnement infrarouge dans le guide d'onde qui ensuite le transporte.

Les moyens d'émission d'un rayonnement infrarouge comprennent des moyens d'émission de chaleur et des moyens susceptibles d'émettre un rayonnement infrarouge sous l'effet de la chaleur émise.

Les moyens d'émission de chaleur sont des moyens résistifs aptes à émettre de la chaleur par effet Joule.

Les moyens d'émission d'un rayonnement infrarouge comprennent une piste résistive en un matériau électriquement conducteur, laquelle est destinée à être reliée à une source d'alimentation en courant électrique, pour émettre de la chaleur et une couche émissive en un matériau susceptible d'émettre un rayonnement infrarouge sous l'effet de la chaleur, ladite couche émissive étant placée entre le guide d'onde et la piste conductrice.

Par ailleurs, les moyens d'émission d'un rayonnement infrarouge sont de préférence en contact direct avec le guide d'onde, de façon à augmenter l'efficacité de la transmission du rayonnement infrarouge dans le guide d'onde.

De préférence, le guide d'onde est au moins partiellement entouré d'un isolant thermique.

Ceci permet encore d'augmenter l'efficacité de la transmission du rayonnement infrarouge à l'intérieur du guide d'onde.

De façon avantageuse, les moyens d'émission d'un rayonnement infrarouge comprennent une couche d'interface en contact avec le guide d'onde.

Dans un mode préféré de réalisation, les moyens d'émission d'un rayonnement infrarouge s'étendent sur ou dans au moins une partie du guide d'onde.

Dans une première variante de réalisation, cette partie est supportée au niveau de son extrémité proximale, son extrémité distale étant libre. L'extrémité distale est dite libre dans la mesure où elle n'est pas supportée par une couche de support. Elle est suspendue au sein d'une cavité.

Dans une deuxième variante de réalisation, les extrémités proximale et distale de la dite partie sont supportées, une cavité étant ménagée entre ces deux extrémités.

Dans un mode préféré de réalisation, la piste conductrice présente une largeur plus faible à proximité de l'extrémité distale de ladite partie que dans le reste du guide d'onde.

De façon avantageuse, la piste conductrice présente une forme effilée en direction de cette extrémité distale.

L'invention concerne également un détecteur de gaz comprenant un dispositif d'émission et de guidage d'un rayonnement infrarouge, conforme à l'invention.

D'autres caractéristiques et avantages de l'invention apparaîtront plus clairement à la lecture de la description qui suit et qui est fait au regard des dessins annexés, sur lesquels :
- la figure 1 est une vue schématique en coupe transversale d'un exemple d'un dispositif d'émission et de guidage selon l'invention,
- la figure 2 représente une étape intermédiaire dans le procédé d'obtention du dispositif illustré à la figure 1,
- les figures 3 à 5 sont des vues de dessus du dispositif illustré à la figure 1, dans le plan I-I, ces figures illustrant différentes variantes de réalisation des moyens d'émission d'un rayonnement infrarouge,
- la figure 6 est une vue en perspective simplifiée du dispositif selon l'invention illustrant son fonctionnement,
- la figure 7 est une vue en coupe transversale d'un autre exemple de réalisation du dispositif d'émission et de guidage d'un rayonnement infrarouge selon l'invention et
- la figure 8 est une vue de dessus dans le plan II-II du dispositif illustré à la figure 7.

Les éléments communs aux différentes figures seront désignés par les mêmes références.

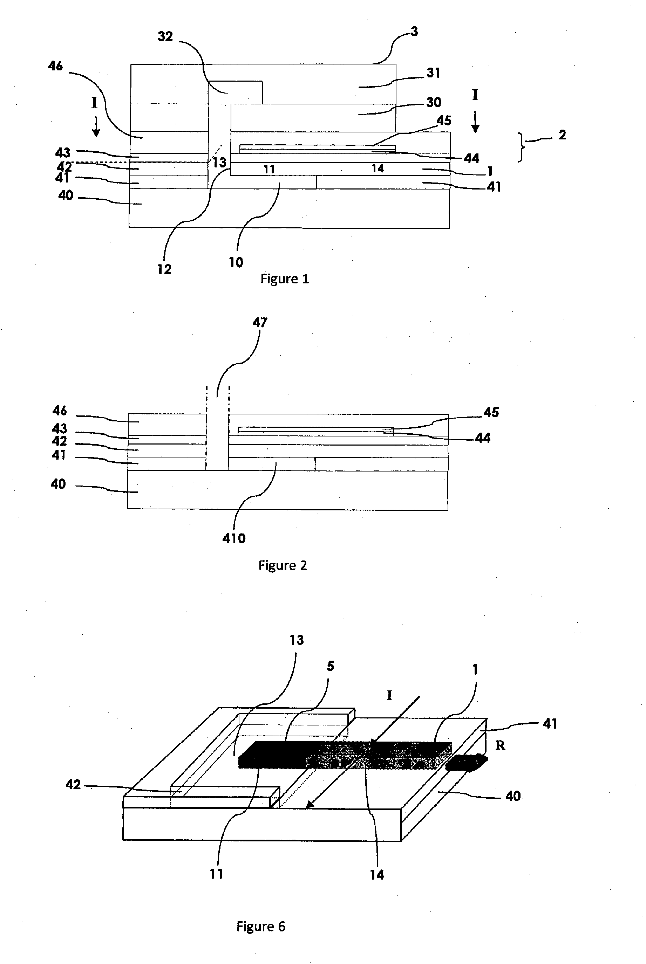

La figure 1 illustre un exemple de réalisation d'un dispositif d'émission et de guidage selon l'invention.

Ce dispositif comporte un guide d'onde 1 sur lequel sont prévus des moyens 2 d'émission d'un rayonnement infrarouge et un capot 3.

La figure 2 illustre plus particulièrement un empilement à partir duquel le guide d'onde et les moyens d'émission d'un rayonnement infrarouge sont obtenus.

Dans l'exemple illustré, cet empilement comporte tout d'abord trois couches : une couche substrat 40 qui est par exemple en silicium, une couche 41 par exemple en silice et une couche 42 par exemple en silicium et, de façon préférée, du SiGe sur silicium.

Les couches 40 et 41 formeront le support du guide d'onde.

De façon générale, l'épaisseur de la couche de support 41 est relativement importante pour éviter les pertes par couplage évanescent, à la longueur d'onde utile, c'est-à-dire sur la plage de longueur d'onde autour de la raie d'absorption du gaz. A titre d'exemple, pour une raie d'absorption de 4,2 µm, la plage est comprise entre 4,1 et 4,3 µm.

A titre d'exemple, l'épaisseur de la couche de support en silice 41 sera d'au moins 1 µm pour une longueur d'onde utile d'environ 4 µm.

L'épaisseur de la couche en silicium 42 pourra être comprise entre 600 nm et plusieurs micromètres.

L'épaisseur de la couche substrat en silicium 40 est supérieure à 80 µm.

Sur la couche 42 est déposée une couche d'interface 43.

Cette couche peut être formée de différents matériaux, notamment SiO₂ ou Si₃N₄, son épaisseur est de l'ordre d'une fraction de la longueur d'onde utile (A), par exemple λ/10.

Par ailleurs, cette couche est facultative. Sa fonction sera expliquée dans la suite de la description.

Sur une partie de l'empilement des quatre couches 40 à 43, est ensuite déposée une couche 44 d'un matériau à bonne émissivité thermique.

Cette couche émissive 44 présente une émissivité thermique, également appelée coefficient d'émissivité, supérieure à 0.5, de préférence supérieure à 0.7, et avantageusement supérieure à 0.9.

On désigne par le terme « *couche émissive* » une couche apte à émettre un rayonnement infrarouge destiné à être transmis dans le guide d'onde.

Cette couche émissive 44 peut notamment être réalisée en TiN.

Sur cette couche émissive 44, est formée une couche résistive 45 en un matériau électriquement conducteur.

Dans une variante de réalisation, la couche émissive 44 est formée sur toute la surface de l'empilement et elle est ensuite gravée pour ne subsister que sur une partie de l'empilement.

La couche résistive 45 peut également être formée sur toute la surface de l'empilement puis gravée avec la couche émissive 44.

Cette couche 45 peut notamment être réalisée en Pt.

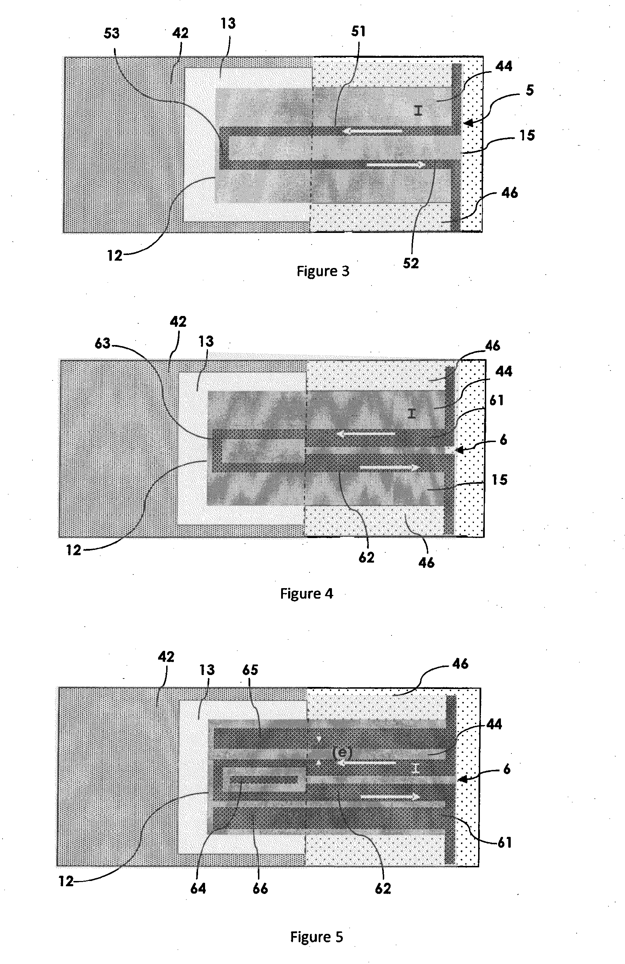

On procède alors à la structuration de la couche résistive 45 de façon à obtenir une piste qui est destinée à être reliée à une source de courant électrique. Cette piste constitue des moyens résistifs susceptibles d'émettre de la chaleur par effet Joule sous l'effet du courant électrique. Cette structuration peut être réalisée grâce à une gravure chimique ionique, des exemples de cette piste seront décrits en référence aux figures 3 à 5.

Lorsque seule la couche 45 est structurée, la couche émissive 44 s'étend de façon continue sur au moins une partie de l'empilement.

Dans une variante, la couche émissive 44 peut également être structurée comme la couche résistive 45. Ainsi, la piste conductrice repose alors sur une couche émissive 44 de même contour.

Enfin, sur l'empilement ainsi obtenu, est déposée une dernière couche de protection 46 qui enrobe complètement les couches 44 et 45, déposées sur une partie de la couche d'interface 43.

Cette couche de protection 46 peut notamment être réalisée en SiO₂ ou Si₃N₄. Comme on le verra dans la suite de la description, cette couche a pour fonction de protéger le dispositif des fuites thermiques et de l'oxydation. Elle pourrait être omise.

De manière générale, les couches 40, 41, 42, 43, 44 et 46 sont réalisées par des techniques de dépôt classique, par exemple un dépôt chimique en phase vapeur (CVD dans la terminologie anglaise), dans lequel un plasma est éventuellement employé (PECVD dans la terminologie anglaise).

Pour le dépôt de la couche résistive 45 en matériau conducteur, une technique de pulvérisation peut être utilisée.

Sur l'empilement ainsi obtenu, est réalisée une étape de lithogravure de façon à obtenir le guide d'onde 1 et, sur celui-ci, les moyens d'émission 2 d'un rayonnement infrarouge.

Le guide d'onde prend alors la forme d'une poutre supportée par les couches 40 et 41, dont l'extrémité porte la référence 12.

Pour une longueur d'onde de 4 µm, la largeur du guide d'onde sera comprise entre 1,5 µm et plusieurs dizaines de microns, voire 100 µm. Sa longueur sera typiquement de l'ordre du millimètre.

Dans l'exemple illustré aux figures 1 et 2, au cours de cette étape de lithogravure, les couches 41, 42, 43 et 46 sont complètement retirées dans la zone 47 délimitée par les deux lignes pointillés sur la figure 2. Cette zone ne traverse pas les couches 44 et 45. Les couches 41, 42, 43 et 46 ont été également retirées sur les côtés longitudinaux de la partie d'extrémité 11 du guide, comme cela est illustré sur les figures 3 à 5. Une cavité 13 est ainsi formée autour du guide d'onde.

De plus, une autre étape est prévue pour éliminer une zone de la couche 41 située sous le guide pour obtenir une cavité 10. Cette zone 410 est délimitée par deux lignes pointillés sur la figure 2. Cette étape est mise en oeuvre grâce, par exemple, à un procédé de gravure chimique haute fréquence.

Grâce à ces différentes étapes, on obtient, à partir de l'empilement des couches 40 à 46, le guide d'onde 1 sur les deux couches de support 40 et 41. Sur le guide, sont formés les moyens 2 d'émission d'un rayonnement infrarouge comprenant les couches 43 et 44, la piste conductrice obtenue à partir de la couche résistive 45 en matériau conducteur et la couche de protection 46.

On comprend donc que les moyens 2 et le guide d'onde 1 constituent un empilement de couches dont certaines sont structurées.

Ainsi, les moyens 2 d'émission d'un rayonnement infrarouge sont en contact direct avec le guide d'onde 1. Ce contact est réalisé entre le guide d'onde 1 et la couche d'interface 43 lorsque celle-ci est prévue ou sinon, entre le guide 1 et la couche émissive 44.

Ainsi, dans l'exemple illustré sur la figure 2, une cavité 10 est prévue sous une partie 11 du guide d'onde. Comme on le verra dans la suite de la description, cette cavité n'est pas nécessaire au fonctionnement du dispositif selon l'invention. Ainsi, la couche de silice 41 pourrait être présente sous l'ensemble du guide d'onde 1.

Il est maintenant fait référence aux figures 3 à 5 qui illustrent trois exemples d'une piste conductrice réalisée à partir de la couche 45 en matériau conducteur.

Toutes ces figures sont des vues de dessus selon le plan I - I de la figure 1.

Ces figures illustrent la partie du guide sur laquelle sont formés les moyens d'émission d'un rayonnement infrarouge et qui est située à proximité de son extrémité.

Cette partie du guide d'onde est partiellement supportée par la couche de support 41. Elle comporte, depuis l'extrémité 12 du guide d'onde, la partie d'extrémité 11 puis une partie 14, illustrées sur la figure 1.

Dans les exemples illustrés sur les figures 3 à 5, la couche 41 a également été retirée sous la partie 11 du guide d'onde la plus proche de l'extrémité 12, comme cela est illustré sur la figure 1.

La cavité 10 ainsi ménagée communique avec la cavité 13 qui est ménagée tout autour de la partie 11 d'extrémité du guide d'onde 1.

Ainsi, dans le mode de réalisation illustré sur les figures, l'extrémité du guide d'onde est isolée thermiquement grâce à l'isolant thermique qui est prévu dans les cavités 10 et 13. Cet isolant thermique peut notamment être constitué par l'air ou le vide ou encore par tout matériau adapté.

Ceci permet d'améliorer l'efficacité de l'émission du rayonnement infrarouge et de sa transmission au guide d'onde.

La couche de support 41 est toujours présente sous la partie 14. Ainsi, la partie du guide d'onde sur laquelle sont prévus les moyens 2 d'émission est libre au niveau de son extrémité distale 12 et maintenue ou supportée, au niveau de son extrémité 15 proximale opposée.

Dans la variante illustrée à la figure 3, la couche résistive 45 en matériau conducteur est structurée pour réaliser une piste 5 en forme de U dont les grandes branches 51 et 52 s'étendent sensiblement parallèlement à la direction longitudinale du guide d'onde, ces deux branches étant reliées par une troisième branche 53 sensiblement parallèle à l'extrémité 12 du guide d'onde.

Cette piste 5 est reliée à une source d'alimentation en courant électrique qui n'est pas illustrée sur la figure 3.

Dans la variante illustrée à la figure 3, les trois branches de la piste 5 présentent sensiblement la même largeur.

Le fonctionnement du dispositif illustré à la figure 3 va maintenant être expliqué, en référence également à la figure 6.

Lorsqu'un courant I passe dans la piste 5, de la chaleur est générée par effet Joule. Cette chaleur diffuse dans la couche émissive 44 réalisée en un matériau à bonne émissivité thermique, ce qui conduit à une émission significative d'un rayonnement infrarouge. Ce dernier est transmis dans le guide. Ce rayonnement infrarouge va ensuite être transporté et guidé, depuis la partie du guide qui est proche de son extrémité 12, vers le reste du guide. Le rayonnement transporté par le guide 1 est symbolisé par la flèche R sur la figure 6. Il est confiné dans le guide d'onde.

De façon générale, le guide d'onde est réalisé en un matériau à faible absorption, de façon à transporter et guider le rayonnement infrarouge sur sa longueur. Ainsi, le guide d'onde n'émet pratiquement pas, de manière intrinsèque, de rayonnement infrarouge.

Par contre, les matériaux déposés sur le guide, en particulier celui de la couche émissive 44, émettent un rayonnement infrarouge qui est ensuite transmis dans le guide et transporté par celui-ci.

Dans les différents modes de réalisation illustrés sur les figures, les moyens d'émission d'un rayonnement infrarouge dans le guide comportent à la fois une piste conductrice, une couche 44 présentant une bonne émissivité thermique, une couche d'interface 43 et une couche de protection 46.

La couche d'interface 43 favorise le couplage entre la couche émissive 44 et le guide d'onde formé à partir de la couche 42, et elle optimise donc la transmission du rayonnement infrarouge depuis la couche émissive 44 vers le guide d'onde 1.

La couche de protection 46 protège le guide d'onde ainsi que les couches émissive 44 et résistive 45 des moyens 2 d'émission, à la fois des fuites thermiques et de l'oxydation.

Cependant, ces deux couches d'interface 43 et de protection 46 pourraient être omises.

La figure 4 illustre une variante de réalisation de la piste conductrice, réalisée à partir de la couche résistive 45 en matériau électriquement conducteur.

Dans l'exemple de ce mode de réalisation, la piste conductrice 6 présente également une forme de U avec deux branches principales 61 et 62 sensiblement parallèles à la direction longitudinale du guide d'onde et une branche 63 les reliant, en étant sensiblement parallèle à l'extrémité 12 du guide d'onde.

Cependant, la largeur des branches 61 et 62 n'est pas constante, contrairement à la variante illustrée à la figure 3. Au contraire, la largeur des branches 61 et 62 est moins importante dans la partie 11 du guide d'onde située au-dessus de la cavité 10 que dans la partie 14 en contact avec la couche de support 41.

La variante illustrée à la figure 4 permet de concentrer, au niveau de la partie 11 du guide d'onde, les pertes par effet Joule générées par la piste conductrice 6, lorsqu'elle est parcourue par un courant électrique.

Ainsi, l'énergie de chauffage émise par effet Joule est concentrée au niveau de la partie 11 du guide qui est, par ailleurs, plus isolée thermiquement grâce à la présence d'un isolant thermique dans les cavités 10 et 13. Ainsi, l'émission du rayonnement infrarouge est également générée au niveau de la partie 11 du guide puis transmise dans celle-ci.

La figure 5 illustre une troisième variante de réalisation, dans laquelle la piste conductrice est identique à la piste 6 illustrée à la figure 4.

Par contre, la couche résistive 45 en un matériau électriquement conducteur a été structurée de façon à conserver, en surface de la couche émissive 44, d'autres zones en matériau conducteur.

Ainsi, du matériau conducteur a été conservé entre les deux branches 61 et 62 de la piste 6, au niveau de la partie 11 du guide, dans laquelle l'épaisseur de ces deux branches est réduite.

Cette zone de matériau conducteur est identifiée par la référence 64.

Par ailleurs, du matériau conducteur est également présent sur la couche émissive 44, au-delà de la piste conductrice 6. Ce matériau forme ainsi deux rubans 65 et 66 qui s'étendent selon la direction longitudinale du guide d'onde. Ces deux rubans sont séparés de la piste conductrice 6 par un espace libre présentant une épaisseur e.

L'intérêt de cette variante de réalisation est de favoriser la diffusion de la chaleur générée par la piste conductrice 6, dans l'ensemble de la partie du guide située à proximité de l'extrémité distale 12. En pratique, le matériau conducteur de la zone 64 et des rubans 65 et 66 permet de diffuser la chaleur générée par la piste 6, lorsqu'elle est parcourue par un courant. La puissance de chauffage reste cependant plus importante dans la partie 11.

Ainsi, l'épaisseur e est choisie la plus faible possible pour optimiser les échanges thermiques par conduction.

L'invention n'est pas limitée aux modes de réalisation qui ont été illustrés en référence aux figures 3 à 5. En particulier, la piste peut également présenter une forme en serpentin ou en double U.

Comme l'illustre la figure 1, le dispositif selon l'invention peut comporter un capot 3.

Ce capot peut être obtenu à partir d'un empilement de deux couches : une couche 30 par exemple en silice et une couche 31 en nitrure de silicium.

Ces deux couches sont partiellement éliminées, notamment grâce à un procédé de lithogravure.

Ceci permet de ménager une cavité 32.

Le capot 3 est placé au-dessus de l'empilement dans lequel ont été formés le guide d'onde et les moyens 2 d'émission d'un rayonnement infrarouge, de telle sorte que la cavité 32 communique avec la cavité 13 ménagée autour du guide d'onde et des moyens 2 d'émission.

Sa fixation peut être assurée par collage, notamment par collage anodique ou moléculaire.

Ce capot 3 contribue à isoler la partie 11 du guide d'onde des processus d'oxydation ou d'échanges thermiques.

Il pourrait également être réalisé à partir d'un substrat de silicium, évidé pour réaliser également une cavité.

Lorsque le capot est réalisé en silicium, il peut être fixé sur la couche 46 de protection grâce à un procédé de scellement par adhérence moléculaire sous gaz rare et à faible conductivité thermique.

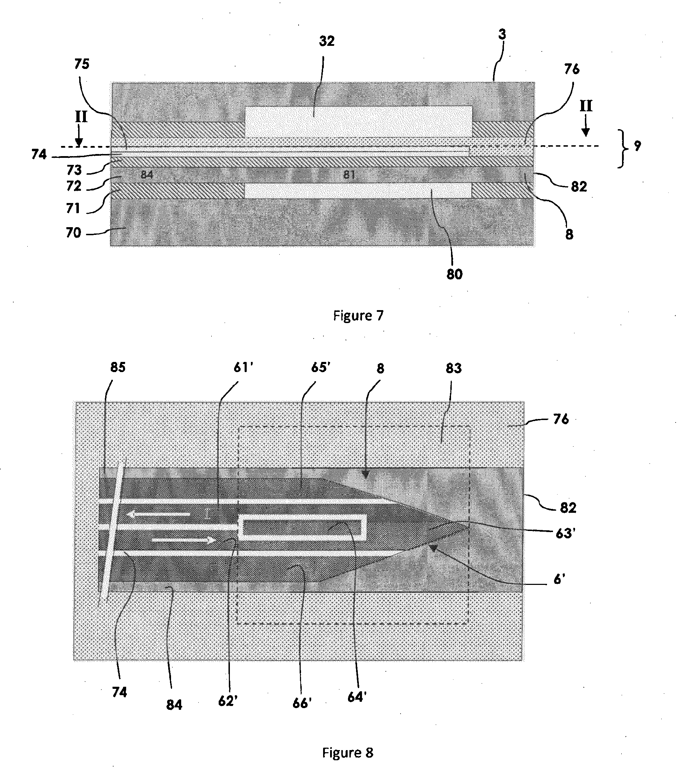

Il est maintenant fait référence aux figures 7 et 8 qui illustrent un autre exemple de réalisation du dispositif d'émission et de guidage d'un rayonnement infrarouge selon l'invention.

Dans ce mode de réalisation, la piste conductrice, réalisée à partir d'une couche en matériau conducteur, est conçue de manière différente.

Le dispositif illustré sur ces figures 7 et 8 est toujours réalisé à partir d'un empilement de plusieurs couches 70 à 76, lesquelles correspondent aux couches 40 à 46 déjà décrites en référence aux figures 1 et 2.

Les matériaux utilisés pour réaliser les couches 40 à 46 peuvent également être utilisés pour réaliser les couches 70 à 76. De même, les mêmes techniques de dépôt peuvent être utilisées.

Par ailleurs, les couches 70 à 76 remplissent les fonctions qui ont déjà été décrites pour les couches 40 à 46.

Enfin, les couches d'interface 73 et de protection 76 sont également des couches facultatives. Cependant, l'efficacité du dispositif est augmentée lorsqu'elles sont présentes.

Comme l'illustre la figure 7, les couches émissive 74 et résistive 75 ne sont réalisées que sur une partie de l'empilement.

Une fois cet empilement obtenu, on procède à la structuration de la couche résistive 75 réalisée en un matériau électriquement conducteur. Cette structuration permet d'obtenir la piste conductrice 6' illustrée sur la figure 8 qui est une vue dans le plan II-II de la figure 7.

Cette structuration peut être réalisée grâce à une gravure chimique ionique.

Par ailleurs, la piste 8 est destinée à être reliée à une source de courant électrique (non représentée).

Après structuration de la couche résistive 75, est déposée une dernière couche de protection 76 qui enrobe complètement les couches émissive 74 et résistive 75 déposées sur une partie de la couche d'interface 73. Cette couche de protection 76 a, comme la couche 46, une fonction de protection contre les fuites thermiques et l'oxydation.

Sur cet empilement des couches 70 à 76, est réalisée une étape de lithogravure de façon à obtenir le guide d'onde 8 et sur celui-ci, les moyens d'émission 9 d'un rayonnement infrarouge.

Le guide d'onde, obtenu à partir de la couche 72, présente la forme d'une poutre qui est supportée par les couches 70, 71.

Sur la partie proche de son extrémité 82, le guide d'onde porte les moyens 9 d'émission d'un rayonnement infrarouge, composés de la couche d'interface 73, de la couche émissive 74 en un matériau à bonne émissivité thermique, de la piste conductrice 6' et de la couche 76 de protection. Dans l'exemple illustré, la couche émissive 74 se trouve uniquement sous la piste conductrice obtenue à partir de la couche résistive 75.

Cette partie du guide d'onde comporte, depuis l'extrémité 82, une partie d'extrémité 81, puis une partie 84.

Dans l'exemple de réalisation illustré sur les figures 7 et 8, après la formation du guide d'onde 8, une autre étape est mise en oeuvre de façon à partiellement éliminer la couche de silice 71 sous la partie 81.

Ainsi, la couche de silice est éliminée sur les côtés longitudinaux de la partie 81. Elle est également partiellement éliminée sous cette même partie 81.

Une cavité 83 est ainsi formée sur les côtés de la partie 81 et elle communique avec la cavité 80 réalisée sous la partie 81. Cette partie 81 est donc libérée par rapport à la couche 70.

En d'autres termes, la partie du guide d'onde sur laquelle sont formés les moyens 9 d'émission est supportée par la couche 71, au niveau de son extrémité distale 82 et de son extrémité proximale 85 opposée.

Comme pour le mode de réalisation illustré aux figures 1 et 2, la partie 81 du guide d'onde peut être isolée thermiquement grâce à un isolant thermique qui est prévu dans les cavités 80 et 83.

Enfin, comme pour le mode de réalisation décrit en référence à la figure 1, un capot 3 peut être prévu de façon à fermer la cavité 83. Ce capot 3 ne sera pas de nouveau décrit.

Il est maintenant de nouveau fait référence à la figure 8 pour décrire la piste conductrice 6' obtenue à partir de la couche résistive 75 en matériau électriquement conducteur.

La piste 6' est une variante de la piste 6 illustrée aux figures 4 et 5.

Ainsi, la piste 6' présente sensiblement une forme de U avec deux branches principales 61' et 62' sensiblement parallèles à la direction longitudinale du guide d'onde et une branche 63' les reliant.

La largeur des branches 61' et 62' est moins importante dans la partie libérée 81 du guide d'onde que dans la partie 84 en contact avec la couche de support 71.

Ceci a pour effet, comme cela a été expliqué au regard de la figure 4, de concentrer l'énergie de chauffage, et donc l'émission du rayonnement infrarouge, au niveau de la partie 81 du guide d'onde qui est la plus isolée thermiquement, grâce à la présence d'un isolant thermique dans les cavités 80 et 83.

Par ailleurs, la partie 63' qui constitue la base du U n'a pas une forme de ruban droit mais une forme de flèche.

Comme illustré à la figure 5, dans ce mode de réalisation, la couche résistive 75 en matériau électriquement conducteur a été structurée pour conserver du matériau conducteur entre les deux branches 61' et 62', au niveau de la partie 81 du guide. Cette zone est identifiée par la référence 64'.

Deux bandes de matériau conducteur 65' et 66' sont également présentes sur la couche émissive 74, au-delà de la piste conductrice 6'.

Ces rubans sont séparés de la piste conductrice 6' par un espace libre présentant une épaisseur e.

Comme expliqué au regard de la figure 5, le matériau conducteur prévu en dehors de la piste 6' permet de favoriser, dans l'ensemble de la partie du guide située proche de son extrémité 82, la diffusion de la chaleur générée par la piste conductrice 6'. Cependant, dans le mode de réalisation illustré, l'énergie de chauffage, et donc l'émission du rayonnement infrarouge, est concentrée au niveau de la partie 81.

La figure 8 montre que l'extrémité des rubans 65' et 66' est biseautée, de façon à être dans la continuité de la partie 63'.

Ainsi, c'est l'ensemble du matériau conducteur présent sur la couche émissive 74 qui présente une forme effilée en direction de l'extrémité distale 82 du guide d'onde.

Ce mode de réalisation permet de rendre plus progressive la transition entre la partie du guide d'onde présentant la piste 6' et celle qui n'est pas parcourue par un courant.

Cette progressivité permet d'éviter des réflexions à la transition entre les deux parties du guide. En effet, ces réflexions sont susceptibles de réduire le rayonnement infrarouge transmis dans le guide d'onde.

Enfin, dans ce mode de réalisation, la partie du guide d'onde présentant les moyens 9 d'émission est supportée à la fois par son extrémité distale et son extrémité proximale.

Ceci permet d'éviter d'éventuelles torsions qui pourraient se produire au niveau du guide d'onde, lors du procédé de réalisation du guide d'onde et des cavités 80 et 83.

Dans l'exemple de réalisation illustré à la figure 8, la longueur de la partie 81 au niveau de laquelle est concentrée l'énergie de chauffage peut être comprise entre 20 µm et 1 mm. Elle est typiquement de 100 µm. Ces valeurs numériques sont également valables pour les exemples de réalisation des figures 3 à 5.

On constate que, dans tous les modes de réalisation qui ont été décrits, les moyens d'émission d'un rayonnement infrarouge s'étendent sur au moins une partie du guide d'onde, située de préférence au niveau d'une extrémité.

Par ailleurs, ces moyens sont en contact direct avec le guide d'onde. En d'autres termes, aucune autre couche ou espace vide n'existe entre les moyens d'émission du rayonnement infrarouge et le guide d'onde.

Enfin, les moyens d'émission du rayonnement infrarouge et le guide d'onde forment un empilement de couches dont certaines sont structurées.

L'invention n'est pas limitée aux modes de réalisation qui viennent d'être décrits.

En particulier, la présence d'une enceinte remplie d'un matériau isolant thermique permet d'augmenter l'efficacité énergétique du dispositif selon l'invention.

Cependant, on obtiendrait une efficacité énergétique acceptable sans cette enceinte, notamment, en prévoyant une couche d'interface avec le guide d'onde qui soit thermiquement isolante. Ceci peut être obtenu avec une couche de silice ou de ZnS-SiO₂ ou encore de nitrure de silicium présentant une épaisseur suffisante, typiquement de quelques microns et, en particulier, supérieure à 1 µm. Par ailleurs, le guide d'onde pourrait être intégralement supporté, sans qu'une cavité soit donc prévue autour d'une partie du guide d'onde.

Dans les différents modes de réalisation qui ont été décrits, les moyens d'émission d'un rayonnement infrarouge sont réalisés sur le guide d'onde et plus particulièrement au-dessus du guide d'onde.

Ces moyens pourraient être encore réalisés sur le guide d'onde mais sur les côtés longitudinaux de celui-ci.

Par ailleurs, ils pourraient également être prévus à l'intérieur du guide d'onde. Dans ce cas, la couche de silicium 42 ou 72 à partir de laquelle le guide d'onde est obtenu, pourrait être réalisée en deux temps.

Une première partie de cette couche serait déposée, les moyens d'émission seraient ensuite formés avant qu'une deuxième partie de cette couche de silicium ne soit déposée.

Par ailleurs, une structuration pourrait être prévue sur le guide d'onde, du même côté des moyens d'émission d'un rayonnement infrarouge mais en aval de ceux-ci, selon la direction R de transport du rayonnement.

Cette structuration pourrait prendre la forme d'une structure à cristaux phoniques ou d'un réseau de Bragg, et pourrait être utilisée pour favoriser l'émission d'un rayonnement infrarouge dans la direction R et l'atténuer dans d'autres directions.

De telles structurations pourraient également être utilisées pour réaliser un filtrage le long du guide d'onde. En effet, le rayonnement infrarouge émis par les moyens d'émission est un rayonnement à large bande. Il pourrait être utile de réaliser un filtrage, en aval de la partie du guide d'onde en contact avec les moyens d'émission, pour extraire un spectre plus étroit.

Enfin, les pistes conductrices décrites en référence aux figures 3 à 5 et 8 peuvent être reliées à une source de courant, par exemple continu.

Cette source de courant électrique pourrait également être modulée pour permettre des modes de détection dynamique.

Ceci peut notamment présenter un intérêt lorsque le dispositif selon l'invention est utilisé dans une application particulière de détection d'un gaz.

En effet, grâce à la présence des moyens d'émission, le guide d'onde transporte un rayonnement infrarouge. Ce rayonnement est susceptible d'exciter un gaz présent autour du dispositif selon l'invention et notamment un gaz présent en aval du dispositif, dans la direction R.

La longueur d'onde du rayonnement infrarouge est alors choisie pour correspondre sensiblement à la longueur d'onde relative à une raie d'absorption du gaz à détecter.

La technique de détection utilisée peut être notamment une technique d'absorption ou une technique de photo-acoustique.

Avec la technique d'absorption, on doit faire interagir le rayonnement transporté par le guide d'onde avec le gaz. Ce peut être fait en interrompant le guide localement pour que la lumière s'y propageant, interagisse avec le gaz et soit absorbée spécifiquement aux longueurs d'onde relatives à une raie d'absorption du gaz. Ce peut être aussi fait en amincissant localement le guide pour que de la lumière se propage autour du guide et « voit » ou « sonde » le gaz pour être partiellement absorbée. Cette zone amincie est typiquement située en dehors du boîtier qui recouvre les moyens d'émission d'un rayonnement infra-rouge.

L'analyse de cette absorption est faite par toute technique d'analyse spectrale. Les techniques fonctionnant sur des principes d'optique guidée (comme l'interféromètre SWIFT) sont particulièrement bien adaptées.

Avec la technique de photo-acoustique, on privilégie l'amincissement du guide et sa libération en structure suspendue. La partie du guide qui est libérée est alors différente de celle comprenant les moyens d'émission d'un rayonnement infrarouge. Dans cette partie du guide à la fois amincie et libérée du substrat, la lumière s'y propageant est forcée d'en sortir un peu pour interagir avec le gaz. Par ailleurs, le guide mis ainsi en forme de poutre peut vibrer sous une excitation qui est liée à la thermalisation des molécules de gaz qui sont excitées par la lumière. Cette excitation peut être modulée par exemple en alimentant en courant la piste conductrice par intervalles, pour solliciter la vibration de la poutre périodiquement et ainsi augmenter la sensibilité de la méthode. On détecte la vibration du guide en forme de poutre par toutes techniques connues, comme un capteur capacitif au voisinage du guide ou des effets piezo électriques, voire un effet optique si l'on conçoit la poutre pour qu'elle comporte une extrémité libérée, en détectant des variations de flux transmis par le guide à sa fréquence de vibration

Les signes de référence insérés après les caractéristiques techniques figurant dans les revendications ont pour seul but de faciliter la compréhension de ces dernières et ne sauraient en limiter la portée.

## Revendications

1. Dispositif d'émission et de guidage d'un rayonnement infrarouge comprenant un guide d'onde et des moyens d'émission d'un rayonnement infrarouge, **caractérisé en ce que** lesdits moyens (2, 9) d'émission d'un rayonnement infrarouge comprennent des moyens d'émission de chaleur sous la forme d'une piste (5, 6, 6') résistive en un matériau électriquement conducteur, laquelle est destinée à être reliée à une source d'alimentation en courant électrique, et des moyens susceptibles d'émettre un rayonnement infrarouge sous l'effet de la chaleur émise sous la forme d'une couche (44, 74) émissive, et sont formés en surface ou à l'intérieur du guide d'onde (1, 8), de telle sorte que ladite couche émissive étant placée entre au moins une partie du guide d'onde et la piste conductrice (5, 6, 6'), le rayonnement infrarouge émis est transmis au guide d'onde qui le transporte.

2. Dispositif selon la revendication 1, **caractérisé en ce que** les moyens (2, 9) d'émission d'un rayonnement infrarouge sont en contact direct avec le guide d'onde.

3. Dispositif selon la revendication 1 ou 2, **caractérisé en ce que** le guide d'onde (1, 8) est au moins partiellement entouré d'un isolant thermique.

4. Dispositif selon l'une des revendications 1 à 3, **caractérisé en ce que** les moyens d'émission d'un rayonnement infrarouge comprennent une couche d'interface (43, 73) en contact avec le guide d'onde (1, 8).

5. Dispositif selon l'une des revendications 1 à 4, **caractérisé en ce que** les moyens (2, 9) d'émission d'un rayonnement infrarouge s'étendent sur ou dans au moins une partie du guide d'onde.

6. Dispositif selon la revendication 5, **caractérisé en ce que** cette partie est supportée au niveau de son extrémité proximale (15), son extrémité distale (12) étant libre.

7. Dispositif selon la revendication 5, **caractérisé en ce que** les extrémités proximale (85) et distale (82) de ladite partie sont supportées, une cavité (80, 83) étant ménagée entre ces deux extrémités.

8. Dispositif selon la revendication 6 ou 7, **caractérisé en ce que** la piste conductrice (6, 6') présente une largeur plus faible à proximité de l'extrémité distale (12, 82) de ladite partie que dans le reste du guide d'onde.

9. Dispositif selon la revendication 8, **caractérisé en ce que** la piste conductrice (6') présente une forme effilée en direction de cette extrémité distale (82).

10. Détecteur de gaz comprenant un dispositif d'émission et de guidage d'un rayonnement infrarouge selon l'une des revendications 1 à 9.

## Patentansprüche

1. Vorrichtung zur Emission und Führung einer Infrarotstrahlung, umfassend einen Wellenleiter und Mittel zur Emission einer Infrarotstrahlung, **dadurch gekennzeichnet, dass** die Mittel (2, 9) zur Emission einer Infrarotstrahlung Mittel zur Emission von Wärme in Form einer resistiven Bahn (5, 6, 6') aus einem elektrisch leitenden Material, welche dazu bestimmt ist, mit einer Quelle zur Versorgung mit elektrischem Strom verbunden zu werden, und Mittel in Form einer emittierenden Schicht (44, 74) umfassen, welche dazu geeignet sind, unter Einwirkung der emittierten Wärme eine Infrarotstrahlung zu emittieren und an der Oberfläche oder im Inneren des Wellenleiters (1, 8) derart ausgebildet sind, dass die emittierende Schicht zwischen wenigstens einem Teil des Wellenleiters und der leitenden Membran (5, 6, 6') positioniert ist, wobei die emittierte Infrarotstrahlung auf den sie transportierenden Wellenleiter übertragen wird.

2. Vorrichtung nach Anspruch 1, **dadurch gekennzeichnet, dass** die Mittel (2, 9) zur Emission einer Infrarotstrahlung im direkten Kontakt mit dem Wellenleiter sind.

3. Vorrichtung nach Anspruch 1, **dadurch gekennzeichnet, dass** der Wellenleiter (1, 8) wenigstens teilweise von einem Wärmeisolator umgeben ist.

4. Vorrichtung nach einem der Ansprüche 1-3, **dadurch gekennzeichnet, dass** die Mittel zur Emission einer Infrarotstrahlung eine Grenzschicht (43, 73) im Kontakt mit dem Wellenleiter (1, 8) umfassen.

5. Vorrichtung nach einem der Ansprüche 1-4, **dadurch gekennzeichnet, dass** die Mittel (2, 9) zur Emission einer Infrarotstrahlung sich auf oder in wenigstens einem Teil des Wellenleiters erstrecken.

6. Vorrichtung nach Anspruch 5, **dadurch gekennzeichnet, dass** dieser Teil auf Höhe seines proximalen Endes (15) gestützt ist, wobei sein distales Ende (12) frei ist.

7. Vorrichtung nach Anspruch 5, **dadurch gekennzeichnet, dass** das proximale (85) und distale (82) Ende des Abschnitts gestützt sind, wobei ein Hohlraum (80, 83) zwischen diesen zwei Enden ausgespart ist.

8. Vorrichtung nach Anspruch 6 oder 7, **dadurch gekennzeichnet, dass** die leitende Bahn (6, 6') nahe dem distalen Ende (12, 82) des Abschnitts eine geringere Breite aufweist als der Rest des Wellenleiters.

9. Vorrichtung nach Anspruch 8, **dadurch gekennzeichnet, dass** die leitende Bahn (6') eine in Richtung dieses distalen Endes (82) zugespitzte Form aufweist.

10. Gasdetektor, umfassend eine Vorrichtung zur Emission und Führung einer Infrarotstrahlung nach einem der Ansprüche 1-9.

## Claims

1. Device for emitting and for guiding infrared radiation comprising a waveguide and means for emitting infrared radiation, **characterized in that** said means (2, 9) for emitting infrared radiation comprise means of emission of heat taking the form of a resistive track (5, 6, 6') made from an electrically-conductive material, which is designed to be connected to an electrical current supply source, and means capable of emitting infrared radiation under the effect of the emitted heat taking the form of an emissive layer (44, 74), and are formed on the surface or inside of the waveguide (1, 8), in such a manner that, said emissive layer being placed between at least part of the waveguide and the conducting track (5, 6, 6'), the infrared radiation emitted is transmitted to the waveguide which transports it.

2. Device according to Claim 1, **characterized in that** the means (2, 9) for emitting infrared radiation are in direct contact with the waveguide.

3. Device according to Claim 1 or 2, **characterized in that** the waveguide (1, 8) is at least partially surrounded by a thermal insulator.

4. Device according to one of Claims 1 to 3, **characterized in that** the means for emitting infrared radiation comprise an interface layer (43, 73) in contact with the waveguide (1, 8).

5. Device according to one of Claims 1 to 4, **characterized in that** the means (2, 9) for emitting infrared radiation extend over or within at least a part of the waveguide.

6. Device according to Claim 5, **characterized in that** this part is supported at its proximal end (15), its distal end (12) being free.

7. Device according to Claim 5, **characterized in that** the proximal end (85) and distal end (82) of said part are supported, a cavity (80, 83) being formed between these two ends.

8. Device according to Claim 6 or 7, **characterized in that** the conducting track (6, 6') has a width that is narrower near to the distal end (12, 82) of said part than in the rest of the waveguide.

9. Device according to Claim 8, **characterized in that** the conducting track (6') exhibits a tapered shape in the direction of this distal end (82).

10. Gas detector comprising a device for emitting and for guiding infrared radiation according to one of Claims 1 to 9.
